(19) Europäisches Patentamt
European Patent Office
Office européen des brevets

(11) Publication number: 0 054 223
A2

# (12) EUROPEAN PATENT APPLICATION

(21) Application number: 81110085.8

(22) Date of filing: 02.12.81

(51) Int. Cl.$^3$: C 12 N 15/00
C 12 N 5/00
//C12R1/91

(30) Priority: 08.12.80 US 213802

(43) Date of publication of application:
23.06.82 Bulletin 82/25

(84) Designated Contracting States:
AT BE CH DE FR GB IT LI NL

(71) Applicant: F. HOFFMANN-LA ROCHE & CO.
Aktiengesellschaft

CH-4002 Basel(CH)

(72) Inventor: Weissbach, Arthur
75 Brookside Terrace
North Caldwell, N.J.(US)

(74) Representative: Mezger, Wolfgang, Dr. et al,
Grenzacherstrasse 124 Postfach 3255
CH-4002 Basel(CH)

## (54) Plasmid vectors for eukaryotic cells and method for selecting transfected eukaryotic cells.

(57) Improved vectors for screening eukaryotic transformants containing heterologous genes. The vectors comprise plasmids containing an insert consisting of a gene coding for an enzyme needed to produce an essential amino acid linked to multiple polyoma virus genes. By growing the transformant eukaryotic cells in a medium missing the essential amino acid corresponding to the vector gene and providing the precursor intermediate in the medium it is possible to screen for cells containing the vector.

EP 0 054 223 A2

Croydon Printing Company Ltd.

RAN 4754/1

# PLASMID VECTORS FOR EUKARYOTIC CELLS AND METHOD FOR SELECTING TRANSFECTED EUKARYOTIC CELLS.

The development of some recombinant DNA plasmid vectors for gene transfer and expression in prokaryotes has been based on the acquisition of cellular antibiotic resistance as the means for selection and maintenance of the recombinant plasmid. See for example U.S. Patent No. 4,190,495 and U.K. Patent No. 2,008,123. An analogous system of selection of biochemical transformation has recently been applied in eukaryotic cell systems. These systems rely on the availability of mutant cell lines, e.g., $tk^-$, $APRT^-$, whose growth characteristics can be altered by the expression of a transferred gene. This limits the use of these eukaryotic cells as hosts for foreign gene expression. The availability of a recombinant DNA plasmid containing a gene which acts as a universal selection method in higher eukaryotic cells would provide a useful general vector.

The present invention takes advantage of the known requirement for essential amino acids which is exhibited by animal cells. L-leucine is one of the eight essential amino acids required by almost all animal species for survival, and one of the 13 amino acids required for mammalian cells to grow in tissue culture. In most cases, this requirement is best explained by the inability of the animal cell to synthesize the amino acid in question, presumably because the necessary enzymatic pathway is not present. This may in turn reflect the absence of the genes

Mez/26.10.1981

coding for the enzymes which comprise the biosynthetic pathway. For some of the essential amino acids, the precursor α-keto acids can replace the corresponding amino acid in the diet. In the case of leucine, α-keto isocaproic acid (the keto acid precursor of leucine) will replace leucine in the diet of young rats, indicating rat cells can transaminate the keto acid to leucine. However, the enzyme catalyzing the penultimate step in leucine biosyntheses, the oxidative decarboxylation of β-isopropyl malic acid (β-IPM) to α-keto isocaproic acid is apparently missing in animal cells. It has been found that rat cells can grow in leucine deficient media containing α-keto isocaproic acid but cannot utilize β-IPM. Therefore, the conversion of β-IPM to α-keto isocaproic acid, which would occur if the gene coding for β-isopropyl malate dehydrogenase could be introduced and expressed in the animal cell, should allow cell growth in the absence of leucine. In lower eukaryotic organisms, such as yeast, the enzyme responsible for this reaction, β-isopropyl malic acid dehydrogenase, is present and the yeast gene that codes for this enzyme (leu 2) has been isolated and cloned in Yep 13 by Broach, et al., Gene 8, 121-133 (1979). Introduction and expression of the yeast leu 2 gene in an animal cell would permit the leucine requirement of the cell to be replaced by β-IPM.

Thus, in one aspect of the invention vectors are prepared which contain within them three functional DNA domains:

1) pBR322 DNA or other equivalent conventional cloning vehicle having multiple drug resistance or other selection sites for propagation of the recombinant plasmids in E. coli,

2) polyoma DNA, so that viral transformed cell growth would act as an alternate phenotypic selection for the vector, and

3) the DNA coding for the enzyme catalyzing the penultimate step in the synthesis of an essential amino acid, such as yeast leu 2 DNA, to provide the gene coding for β-isopropyl malic acid dehydrogenase. In a preferred embodiment the vector contains two polyoma DNA molecules joined in tandem, head-to-tail.

The general technique of selection, using the precursor [β-isopropyl malic acid] in place of the amino acid [leucine] can be applied in similar manner to any of the other essential amino acids; tryptophan, phenylalanine, lysine, threonine, valine, methionine, and isoleucine. In addition, histidine is an essential amino acid in almost all species except humans. Using an analogous approach the selection procedure for these amino acids would involve substituting the penultimate precursor to replace the given amino acid in the culture media, i.e.:

a) indole glycerol phosphate (or indole and serine) in place of tryptophan
b) histidinol (or imidazole glycerol phosphate) for histidine
c) prephenic acid for phenylalanine.

<u>The Construction of pBR322-Polyoma DNA - Yeast LEU 2 Recombinant Plasmids</u>

The starting plasmid was pBR322.A pBR322-polyoma-polyoma (RI) recombinant, which we designate pBR-PY-PY, was isolated from a stock provided by M. Martin, NIH, Bethesda, Md., that was reported to contain a recombinant with only a single polyoma insert (Israel, M.A., Chan, H.W., Rowe, W.P., and Martin, M.A., Science <u>203</u>, 883-887 [1979]). The source of the LEU2 gene of yeast was YEp13, a plasmid provided by J. Broach, Cold Spring Harbor, N.Y. and propagated in E. coli HB 101 in L. Broth with 20 µg/ml ampicillin (Broach, J.R., Strathern, J.N., and Hicks, J.B., Gene <u>8</u>, 121-133 [1979]).

pBR-PY-PY was cleaved with restriction endonuclease SalI at the SalI site within the tetracycline resistance region. All endonucleases restrictions were performed as recommended by the vendor. Thus, pBR-PY-PY (2 μg) was treated with 3 units of SalI restriction endonuclease at 37°C for 15 minutes and the procedure repeated once. The Sal I linearized plasmid was then treated with bacterial alkaline phosphatase using 0.4 units/μg DNA at 37°C for 30 minutes.

Yeast leu 2 DNA was isolated from YEp13 (50 μg) by cleavage of the plasmid with restriction endonucleases SalI (65 units) at 37°C for 30 minutes and the procedure was subsequently repeated once. After preparative electrophoresis in 1% agarose, small strips of the preparative gel were stained in 1 μg/ml of EtBr to determine the position of the 2.2 kb fragment. The remainder of the gel was sliced at this point and mashed with 3 vol 0.87 M NaCl/1% Sarkosyl in 1.5 ml Eppendorf tube to soak elute the 2.2 kb fragment containing the leu 2 sequence. The DNA was eluted by mixing overnight at 4°C. After centrifugation, the supernatant was diluted with 2 vol buffer A (150 mM Tris, pH 8.0, 6 mM EDTA) and made 50% (v/v) in ethanol. This solution was applied to a CF-11 cellulose column and the DNA was isolated from the column by elution with an aqueous buffer.

Ligation of the linearized pBR-PY-PY to the LEU2 gene was performed with 2 μg of pBR-PY-PY and 0.67 μg of LEU2 with 2 units of T4 ligase at 15°C for 18-20 hrs. The ligation mixture was diluted as needed to transfect E. coli RRI as described below.

E. coli RRI was grown in L. Broth to mid-log phase to $5.2 \times 10^7$ cells/ml. The cells, $6.5 \times 10^8$, were transfected with 30 ng, 50 ng or 70 ng of the DNA in the ligation mix. Transfected cells were plated and selected for ampicillin resistance in media containing 200 μg/ml of ampicillin.

Ampicillin resistant clones were screened for tetracycline sensitivity in media containing 12.5 μg/ml of tetracycline and those clones that were $Amp^r Tc^s$ were isolated. These were screened for yeast LEU2 sequences by the rapid alkaline isolation method of Birnboim, H.C. et al., Nucleic Acids Research 7, 1513-1523 (1979). DNA was transferred from 1% agarose gels to BA-85 nitrocellulose sheets and hybridized to nick translated $^{32}P$-labeled leu 2 DNA by the method of Southern, E.M., Journal of Molecular Biology 98, 508-517 (1975).

The LEU2 positive clones were propagated in ampicillin containing media and the plasmid DNA isolated as stated above. Each clone was mapped by restriction enzyme analysis and Southern hybridization to determine the orientation of the inserted leu 2 sequence(s). Recombinant DNA plasmids containing the LEU2 gene inserted into pBR-PY-PY are code named "leupy".

Transfection of Fischer rat cells by Leupy 4

Fischer rat kidney cells ($3 \times 10^5$) were placed in 60 mM dishes and grown overnight. Calcium phosphate precipitates of Leupy clones 4A1-507 and 4B1-538 and salmon sperm carrier DNA were prepared according to Wigler et al., Cell 16, 777-785 (1979) and the cells were transfected for 3 hrs according to the procedure of Graham, F.L. and Van der Eb, A.J., Virology 52, 456-467 (1973). After the transfection process, cells were grown for an additional 48 hours prior to selection in soft agar. The cells were then trypsinized and plated at $1 \times 10^5$ cells per 35 mm dish in 0.33% agar in DME (Dulbecco's modified Eagle medium) over a 0.5% agar in DME feeder layer. After 15 days, colonies of cells were removed from the soft agar and plated at low density (100-500 cells per 60 mM plate) to allow for the formation of cell foci. After 8 days, one focus was removed from each plate and established as a cell line. Controls were transfected only with polyoma DNA and transformed colonies were isolated in soft agar.

Leucine Requirement of Rat Cells

The Fischer Rat Kidney Cell line used in these studies will not grow in media lacking L-leucine, but will grow in leucine$^{(-)}$ media if α-keto isocaproic acid is present, or if leucine is added back. Rat cells, after exposure to leucine$^{(-)}$ media for 14 days are not longer viable. It has been found that murine 3T3 or 3T6 cells exhibit the same nutritional characteristics. Control experiments show that growth in the leucine(-) media, after resupplementation with leucine, proceeds as in normal DME medium.

The concentration of L-leucine in DME media (GIBCO) is 0.8 mM; addition of β-isopropyl malic acid at this concentration, or of the β-IPM racemate at 1.6 μM will not inhibit cell growth in DME media, showing that β-IPM is not toxic to cells. Further controls using Fischer rat cells transformed by polyoma DNA, as isolated in this laboratory, show the transformed cells also are unable to grow in leucine deficient media in the absence or presence of β-IPM.

Structure of pBR322-Polyoma-LEU2 Recombinant Plasmid

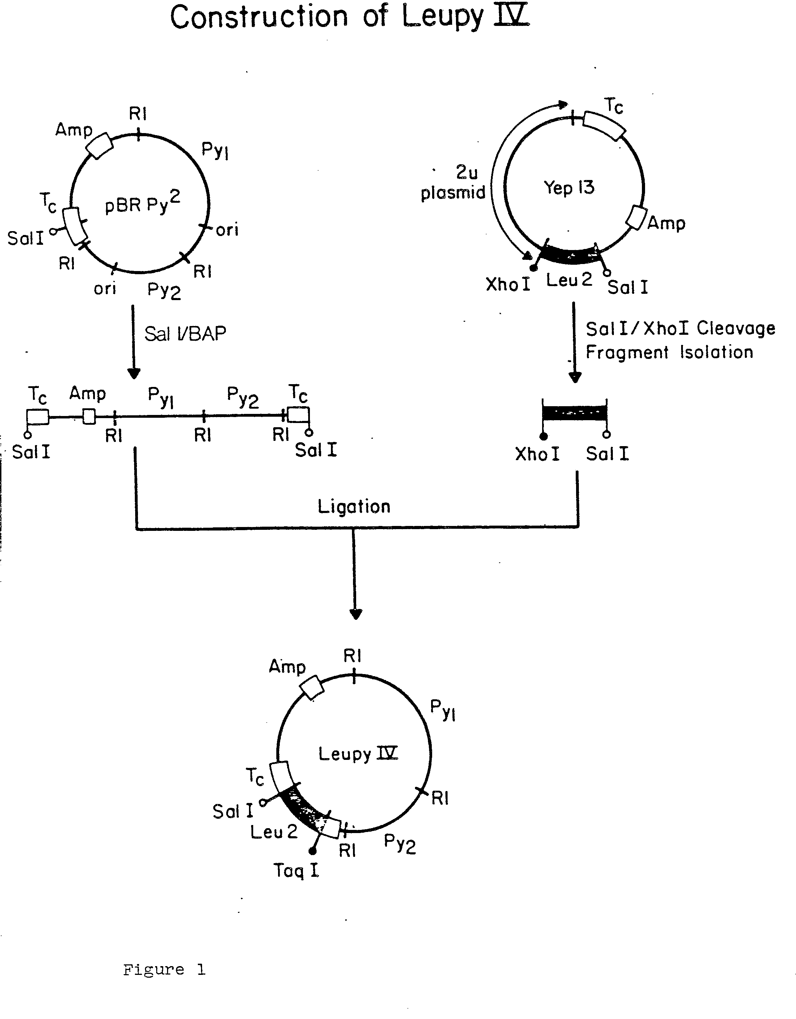

Analysis of the pBR322-polyoma recombinant DNA supplied by Dr. Malcolm Martin (NIH) revealed that the particular clone isolated contained a tandem head-to-tail repeat of the polyoma DNA inserted into the EcoRI site of pBR322. The fragments produced by the action restriction endonucleases BamHI, Hind III and SSTI on this plasmid were determined. BamHI digestion of the pBR322-polyoma DNA recombinant releases an intact 5.3 kb polyoma genome. Since BamHI makes a single cut in polyoma DNA, generation of polyoma sized DNA would only arise from a head-to-tail repeat of the polyoma DNA inserted into pBR322 as represented in Figure 1. This figure also diagrams the construction of pBR322-PY DNA-yeast 2 leu recombinant (leupy). The yeast LEU2 gene, contained in a DNA fragment isolated from the plasmid YEp13 by the action of SalI and XhoI restriction endonucleases, was ligated to the pBR322-PY-PY plasmid at the SalI site. The recombinant plasmid, leupy 4, contains the yeast LEU2 gene inserted into the tetracycline region of pBR-PY-PY at the SalI site and contains the initial SalI site and a new TaqI site (generated by the Xho-Sal complementary ends) at the ends of the LEU2 gene insert.

After transfection and growth in the E. coli RRI four different leupy recombinants were obtained as determined by restriction endonucleases analysis data. Clone 4B1 and 4B2 have identical EciRI restriction maps except for the generation of an additional 2.2 kb fragment (corresponding to a fully intact leu 2 fragment) from clone 4B2. Similar results were obtained with clones 4A1 and 4A2. All four clones exhibit a single 5.3 kb band that corresponds to 2 copies of full length polyoma DNA which was originally inserted by EcoRI cleavage into pBR322. Orientation of the leu 2 inserts was determined from the remaining fragments in the digest.

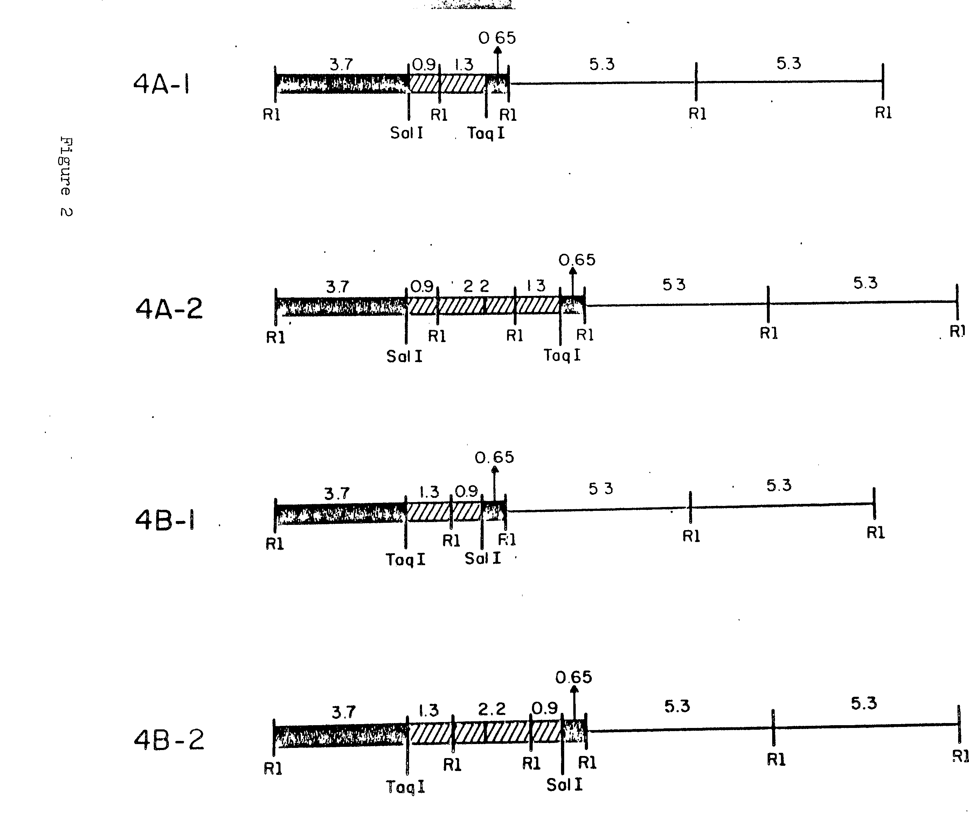

Based on this data it was concluded that the structure of the four leupy recombinants are those shown in Figure 2. Leupy 4 A-1 and 4 B-1 contain the yeast LEU2 gene as a single copy in opposite orientations. Leupy 4 A-2 and 4 B-2 contain the LEU2 DNA as a tandem head-to-tail repeat in either of the two possible orientations. In the 4A clonal series the 4.6 kb fragment corresponds to the large portion (3.7 kb) of pBR322 from the RI site to the SalI site plus the 0.9 kb fragment SalI/EcoRI fragment of the LEU2 DNA. The 0.9 kb fragment has been reported to contain the region coding for the β-isopropyl malic acid dehydrogenase. Likewise, the 5.0 kb fragment produced by RI digestion of the 4B clonal series was indicative of a

BAD ORIGINAL

reverse orientation whereby the 3.7 kb pBR322 fragment was ligated to the large 1.3 kb XhoI/RI fragment of LEU2.

In an alternative embodiment a polyoma DNA-polyoma DNA dimer is constructed by use of procedures known in the art and then inserting the dimer into the commercially available pBR325 plasmid. The pBR325 plasmid is distinguished in having one additional selection site (chloramphenicol resistance) to those found in pBR322.

It should also be noted that a series of earlier attempts to make polyoma-leu 2 recombinants linked to E. coli plasmids were unsuccessful. These attempts included:

1) The joining of a BamHI/HincII cleaved polyoma DNA to pYeleu 10, a recombinant plasmid of ColE1 attached to the yeast LEU2 gene (Ratzkin and Carbon, PNAS 74, 487-491 [1977]).

2) The joining of a BamHI cleaved polyoma DNA to a BamHI cleaved polyoma DNA to a BamHI treated pYeleu 10.

3) The joining of a BamI cleaved polyoma DNA directly to the BamHI site of Yep13.

CLAIMS:

1. A selectable recombinant DNA plasmid vector for transfection of eukaryotic cells comprising three functional DNA domains consisting of (1) a conventional cloning vehicle having multiple selection sites, (2) polyoma DNA and (3) DNA coding for an enzyme catalyzing one of the steps in the biosynthesis of an essential amino acid required for eukaryotic cell growth.

2. The selectable recombinant DNA plasmid vector of claim 1 wherein said conventional cloning vehicle is pBR322.

3. The selectable recombinant DNA plasmid vector of claim 1 wherein said polyoma DNA is present as a dimer in head-to-tail configuration.

4. The selectable recombinant DNA plasmid vector of claim 1 wherein the enzyme DNA is the yeast LEU2 gene.

5. A method for selecting transfected eukaryotic cells which method comprises providing the selectable recombinant DNA plasmid vector of claim 1, transfecting eukaryotic cells with said vector and growing said cells in a medium deficient in one of the amino acids required for eukaryotic cell growth but supplemented with the substrate compound for said enzyme.

6. The method of claim 5 wherein the selectable recombinant DNA plasmid vector is that of claim 2 or claim 3.

7. The method of claim 5 wherein the enzyme DNA in said selectable recombinant DNA plasmid vector is the yeast LEU2 gene and said substrate compound is β-isopropyl malic acid.

8. A eukaryotic cell transfected with a selectable recombinant DNA plasmid vector as claimed in any one of claims 1-4.

***

Claims for Austria:

1. A method for preparing transfected eukaryotic cells which process comprises transfecting eukaryotic cells with a selectable recombinant DNA plasmid vector containing three functional DNA domains consisting of (1) a conventional cloning vehicle having multiple selection sites, (2) polyoma DNA and (3) DNA coding for an enzyme catalyzing one of the steps in the biosynthesis of an essential amino acid required for eukaryotic cell growth.

2. A method as claimed in claim 1 wherein said conventional cloning vehicle is pBR322.

3. A method as claimed in claim 1 wherein said polyoma DNA is present as a dimer in head-to-tail configuration.

4. A method as claimed in claim 1 wherein said enzyme DNA is the yeast LEU2 gene.

5. A method for selecting transfected eukaryotic cells which method comprises providing the selectable recombinant DNA plasmid vector of claim 1, transfecting eukaryotic cells with said vector and growing said cells in a medium deficient in one of the amino acids required for eukaryotic cell growth but supplemented with the substrate compound for said enzyme.

6. The method of claim 5 wherein the selectable recombinant DNA plasmid vector is that of claim 2 or claim 3.

7. The method of claim 5 wherein the enzyme DNA in said selectable recombinant DNA plasmid vector is the yeast LEU2 gene and said substrate compound is β-isopropyl malic acid.

***

# Construction of Leupy IV

Amp
RI
Py1
Tc
pBR Py2
Sal I
ori
RI
RI
ori
Py2
Sal I/BAP
Tc
2u
plasmid
Yep 13
Amp
Xho I
Leu 2
Sal I
Sal I/XhoI Cleavage
Fragment Isolation
Tc
Amp
Py1
Py2
Tc
Sal I
RI
RI
RI
Sal I
Xho I
Sal I
Ligation
Amp
RI
Py1
Leupy IV
Tc
Sal I
Leu 2
RI
RI
Py2
Taq I


Figure 1

2|2

4A-1
3.7
0.9
1.3
0.65
5.3
5.3
R1
Sal I
R1
Taq I
R1
R1
R1
4A-2
3.7
0.9
2 2
1 3
0.65
5 3
5.3
R1
Sal I
R1
R1
Taq I
R1
R1
R1
4B-1
3.7
1.3
0.9
0.65
5 3
5.3
R1
Taq I
R1
Sal I
R1
R1
R1
4B-2
3.7
1.3
2.2
0.9
0.65
5.3
5.3
R1
Taq I
R1
R1
Sal I
R1
R1
R1

Figure 2